# FASCICULE DE BREVET EUROPEEN

(11) **EP 0 729 743 B1**
(45) Date de publication et mention de la délivrance du brevet: **23.05.2001**
(21) Numéro de dépôt: 96400433.7
(22) Date de dépôt: 29.02.1996
(51) Int. Cl.: A61K 7/13

(54) **Composition colorante pour cheveux contenant un broyat de plantes ou de parties de plantes de l'espèce Impatiens balsamina, et son application**
Haarfärbemittel, enthaltend zerdrückte Impatiens-Balsamina-Pflanzen oder -Pflanzenteile, und dessen Verwendung
Hair dyeing composition comprising crushed plants or plant parts of impatiens balsamina and its application

(30) Priorité: 01.03.1995 FR 9502361
(43) Date de publication de la demande: 04.09.1996
(73) Titulaire: L'OREAL, 75008 Paris (FR)
(72) Inventeur: Belcour-Castro, Béatrice, 37000 Tours (FR); Martin, Richard, 37210 Roche Corbon (FR)
(74) Mandataire: Tonnellier, Jean-Claude

(56) Documents cités:
- EP-A- 0 124 393
- DATABASE CHEMABS CHEMICAL ABSTRACTS SERVICE, COLUMBUS, OHIO, US AN: 90:88642, M.A: MIKAILOV ET AL: "Balsam culture as a valuable dye" XP002004902
- DATABASE CHEMABS CHEMICAL ABSTRACTS SERVICE, COLUMBUS, OHIO, US AN: 115:73525, RAMA S. JAHAN ET AL: "Balsam flowers, useful in wool dyeing" XP002004903
- DATABASE BIOSIS BIOSCIENCES INFORMATION SERVICE, PHILADELPHIA, PA, US AN: 79:170369, M. THAKUR ET AL: "Antho Cyanin pigmentation in roots of impatiens-SPP" XP002004904

## Description

L'invention a pour objet une composition colorante pour cheveux, ainsi qu'un procédé de coloration des cheveux à l'aide d'une telle composition.

Plus précisément, l'invention concerne une composition colorante comprenant comme agent colorant un broyat d'au moins un organe de l'espèce végétale *Impatiens balsamina.*

Pour colorer les cheveux, on utilise actuellement de nombreux colorants, dont la plupart sont d'origine synthétique, qui sont soit des colorants directs, soit des colorants d'oxydation (dont le pouvoir colorant ne se développe qu'en présence d'un agent oxydant).

L'application de colorants synthétiques peut provoquer chez certaines personnes des phénomènes d'allergie. Il est bien évident que pour ces personnes, il est souhaitable d'éviter l'utilisation de tels colorants synthétiques.

C'est une des raisons pour lesquelles on observe actuellement une grande faveur pour les produits colorants d'origine végétale, avec lesquels les réactions allergiques sont plus rares.

Un des matériaux végétaux les plus utilisés pour la coloration des cheveux est la poudre de henné qui résulte du broyage de feuilles séchées du végétal *Lawsonia inermis*. Toutefois, les propriétés colorantes du henné varient notamment en fonction de l'origine de la plante ou de l'époque de la récolte.

Il est donc important de pouvoir disposer de matériaux colorants d'origine végétale ayant un pouvoir colorant constant et aisément reproductible.

On a maintenant découvert que les broyats d'au moins un organe du végétal *Impatiens balsamina* constituent des agents colorants particulièrement intéressants, en particulier lorsqu'ils sont appliqués sur les cheveux sous la forme d'une dispersion dans un véhicule liquide, ladite dispersion étant suffisamment épaisse pour pouvoir enrober les cheveux et y adhérer pendant le temps nécessaire au développement de la coloration.

L'article de Mikailov et al, Dok. Akad. Nauk. Az. SSR (1978), 34 (8), pages 72-75 décrit l'utilisation d'un broyat de feuilles d'Impatiens balsamina dispersé dans l'eau pour teindre la laine.

L'invention a donc pour objet une composition colorante pour cheveux, sous forme de poudre, comprenant comme agent colorant un broyat de plantes ou parties de plantes de l'espèce *Impatiens balsamina*. Le broyat peut être en particulier obtenu au départ de racines, de feuilles, de fleurs, des parties aériennes de la plante (c'est-à-dire la plante entière moins les racines), ou encore au départ de la plante entière.

Selon un mode de réalisation particulier, le broyat est au moins partiellement déshydraté, ce qui facilite notamment sa conservation.

L'agent colorant peut être obtenu notamment par lyophilisation des plantes ou parties de plantes et broyage.

Les compositions de l'invention peuvent être préparées notamment sous la forme de poudres sèches, de façon à permettre leur bonne conservation. On peut ainsi par exemple lyophiliser les plantes ou parties de plantes (préalablement pulvérisées ou non) pour obtenir un produit dont la masse représente moins de 10 %, et en particulier moins de 5 %, de la masse de plantes (ou parties de plantes) fraîches de départ, puis, le cas échéant, on broie le lyophilisat obtenu.

Les dimensions des particules de broyat, lyophilisé ou non, sont par exemple dans une gamme allant de 0,1 à 2000 µm, notamment de 0,5 à 750 µm, et en particulier de 1 à 500 µm.

La composition colorante de l'invention peut bien entendu contenir d'autres ingrédients ou excipients usuels tels que des agents épaississants (par exemple gomme, pectine, alginate), des agents tensioactifs, des agents anti-oxydants, des agents de pénétration, des agents séquestrants, des parfums, des tampons ou modificateurs de pH, des agents dispersants, des agents de traitement ou de conditionnement des cheveux, des agents filmogènes, des agents conservateurs, des agents opacifiants. Ces autres ingrédients sont ajoutés sous forme de poudres ou sous forme de solutions d'imprégnation du broyat (par exemple des solutions dans un solvant volatil qui est ensuite éliminé). La composition peut également contenir d'autres agents colorants et notamment d'autres colorants végétaux, soit sous forme d'extraits soit sous forme de poudres provenant du broyage de plantes ou de parties de plantes contenant un agent colorant. Le broyat d'*Impatiens balsamina* représente généralement de 2 à 100 % de la masse totale de la composition sous forme de poudre.

Il est possible de cultiver facilement *in vitro* certains organes *d'Impatiens balsamina*, notamment les racines de cette plante. Des conditions permettant d'effectuer ces cultures *in vitro* sont données ci-après dans la partie expérimentale. Il est ainsi possible, en employant des conditions de culture constantes, déterminées préalablement par des expériences de routine, d'obtenir un agent colorant ayant des propriétés constantes quelle que soit l'époque de préparation.

Pour l'application sur les cheveux, la composition de l'invention est mise sous la forme d'une dispersion du broyat dans un véhicule liquide approprié, ladite dispersion ayant une consistance suffisante pour adhérer aux cheveux sans s'écouler après application sur lesdits cheveux.

L'invention a également pour objet un procédé de coloration des cheveux
caractérisé par le fait qu'il comprend les étapes consistant à :
a) obtenir une composition sous la forme d'une dispersion aqueuse contenant un broyat de plantes ou de parties de plantes de l'espèce *Impatiens balsamina*, dans un véhicule liquide approprié, ladite dispersion ayant une consistance suffisante pour enrober les cheveux et y adhérer sans s'écouler après l'application sur lesdits cheveux ;
b) à mettre en contact les cheveux à colorer avec une composition telle qu'obtenue en a) ci-dessus ;
c) et à maintenir ledit contact pendant un temps suffisant pour obtenir la coloration désirée.

La dispersion aqueuse obtenue à l'étape a) ci-dessus est appliquée sous forme de cataplasme enrobant les cheveux. La proportion pondérale du matériau colorant (broyat), en poids de matière sèche, est généralement dans la gamme de 1 à 50 %, et en particulier de 3 à 40 %, en poids par rapport au poids total de la composition. Si désiré, on peut augmenter la consistance de la composition par addition d'un agent épaississant usuel, tel qu'une gomme, une pectine, un alginate, etc... La composition prête à l'emploi a généralement une viscosité allant 0,5 à 4,5 Pa.s (5 à 45 poises), par exemple de 0,8 à 3,5 Pa.s (8 à 35 poises). Le véhicule liquide utilisé pour obtenir la composition prête à l'emploi est un véhicule aqueux, notamment de l'eau ou un mélange d'eau et d'un solvant compatible avec l'application sur les cheveux et le cuir chevelu (par exemple un alcool inférieur comme l'éthanol).

On peut en outre ajouter à la composition, au moment de l'emploi, des excipients ou des adjuvants usuels en cosmétologie capillaire tels que par exemple des modificateurs de pH, des agents de traitement ou de conditionnement des cheveux, des agents mouillants, des agents épaississants, etc... Le pH de la dispersion aqueuse, prête à l'emploi, est généralement compris entre 2,5 et 12, de préférence entre 3 et 10,5.

Le temps de contact de la composition colorante de l'invention avec les cheveux dépend notamment de la coloration initiale des cheveux et de la nuance que l'on désire leur conférer. Les temps de contact peuvent être déterminés de façon systématique par de simples expériences de routine sur des échantillons de cheveux. Ils peuvent varier par exemple de 5 minutes à 4 heures, en particulier entre 15 minutes et 1 heure. La composition est appliquée sur la chevelure, et on peut appliquer des moyens de chauffage usuels, pour opérer à une température ne dépassant pas 45°C. On peut aussi opérer sans moyens de chauffage, à température ambiante, ou encore avec une eau légèrement tiédie.

Après le temps de contact désiré, on rince les cheveux de façon à éliminer notamment les résidus solides de la composition appliquée.

Sur des cheveux blancs, la composition colorante de l'invention fournit, dans le cas d'un broyat de racines d'*Impatiens balsamina*, une coloration blond cuivré, et sur des cheveux blonds, on obtient des reflets cuivrés. Avec d'autres parties de la plante, on peut obtenir diverses nuances pouvant aller par exemple d'un brun jaunâtre moyen à un brun puissant (voir la partie expérimentale ci-après).

L'application des compositions de l'invention à des sujets volontaires a montré que ces compositions n'ont pas d'effet irritant ni d'effet allergisant.

En outre, les colorations obtenues présentent une bonne tenue et, en particulier, résistent bien aux shampooings et à la sueur.

Les exemples suivants illustrent l'invention.

### EXEMPLE 1 : Culture de racines d'Impatiens balsamina

Des fragments de feuilles d'*Impaliens balsamina* ont été mis en culture, sur un milieu gélosé (milieu A) stérile dont la composition sera donnée ci-après, dans les conditions suivantes : des feuilles d'*Impatiens balsamina* sont immergées dans une solution aqueuse d'hypochlorite de calcium à 40 g/l pendant 15 minutes afin de procéder à leur décontamination. Les feuilles sont ensuite rincées par 4 passages successifs dans de l'eau stérile. Les feuilles sont ensuite découpées en fragments d'environ 0,5 cm² puis sont placées sur milieu A gélosé à raison de deux fragments par boîte. Les cultures sont maintenues à 26°C à l'obscurité.

De façon constante, on observe que certains de ces fragments de feuilles donnent naissance à des chevelus racinaires. Ces chevelus racinaires ont ensuite été transférés en milieu A liquide (sans gélose) stérile.

Les racines ainsi obtenues sont entretenues dans ce milieu liquide, à l'obscurité et sous agitation (100 rotations/min) à la température de 26°C.

Le repiquage peut être effectué par exemple toutes les deux semaines. Pour cela, des racines sont prélevées et reprises pour inoculer, à raison de 25 g/l, du milieu A neuf, ce milieu neuf étant préalablement réparti en fioles d'Erlenmeyer de 250 ml à raison de 100 ml par fiole et stérilisé pendant 20 minutes à 115°C.

A l'issue de plusieurs cycles d'entretien, certaines souches ont présenté une diversification phénotypique mise en évidence par la couleur des racines : certaines d'entre elles apparaissent colorées en rose, alors qu'à l'origine, les racines avaient une couleur jaune pâle. Ces racines ont été séparées et multipliées. On a ainsi obtenu diverses souches de racines d'*Impatiens balsamina* et en particulier une souche Ib1 (non colorée en rose) et Ib2 (ayant une légère coloration rose).

Des racines d'*Impatiens balsamina* issues de cultures âgées de 2 semaines ont été lyophilisées.

Pour cela, on prélève les tissus racinaires et on les lyophilise après congélation.

Pour 25 g de matière fraîche (racines) on obtient environ 1 g de lyophilisat.

Le lyophilisat est réduit en poudre fine par broyage.

Les dimensions des particules de la poudre sont de l'ordre de 1 à 2 µm.

Le milieu A utilisé avait la composition suivante :

| *Constituants* | *Concentrations (mg.l*^{*-1*}*)* |
|---|---|
| KNO₃ | 1900,000 |
| NH₄NO₃ | 1650,000 |
| KH₂PO₄ | 170,000 |
| MgSO₄, 7H₂O | 370,000 |
| CaCl₂, 2H₂O | 440,000 |
| MnSO₄, 4H₂O | 22,300 |
| H₃BO₃ | 6,200 |
| ZnSO₄, 7H₂O | 8,600 |
| Na₂MoO₄, 2H₂O | 0,250 |
| CuSO₄, 5H₂O | 0,025 |
| CoCl₂, 6H₂O | 0,025 |
| KI | 0,830 |
| FeSO₄, 7H₂O | 27,850 |
| Méso-inositol | 100,000 |
| Acide nicotinique | 0,500 |
| Pyridoxine, HCl | 0,500 |
| Thiamine, HCI | 0,100 |
| L-glycine | 2,000 |
| Na₂EDTA | 37,300 |
| Acide naphtylacétique (ANA) | 1,000 |
| Kinétine | 0,060 |
| Saccharose | 30000,0 |

Le pH est ajusté à 5,8 avant stérilisation.

Pour les milieux A gélosés, on ajoute de l'agar à 8 g/l.

### EXEMPLE 2 :

On mélange 1,5g de poudre de lyophilisât obtenue à l'exemple 1 avec 10 ml d'eau pour former une pâte. La pâte ainsi obtenue est appliquée sous forme de cataplasme sur la moitié de la longueur d'une mèche de cheveux blancs (du côté de la racine des cheveux).

L'application est maintenue pendant 3 heures 30 min. à température ambiante. Les cheveux sont ensuite rincés abondamment à l'eau courante pour éliminer la pâte colorante, puis ils sont séchés.

Avec les poudres provenant des souches Ib1 et Ib2, les cheveux ainsi traités ont une coloration blonde avec des reflets cuivrés. La coloration est régulière.

Avec certaines souches de culture de racines, on peut observer de légères différences dans les tonalités de la coloration obtenue, avec une nuance plus rose.

Une même culture établie de racines donne des produits dont les propriétés colorantes restent constantes par application sur des cheveux semblables pendant un temps donné.

Dans d'autres expériences, l'application sur les cheveux a été maintenue pendant 30 minutes au lieu de 3 heures 30 minutes. L'intensité de la coloration est alors légèrement plus faible.

Il est donc possible, en fonction de la couleur d'origine des cheveux, de régler l'intensité de la coloration jusqu'au degré souhaité en fonction du temps d'application. On peut déterminer préalablement, par des expériences de routine effectuées sur des mèches de cheveux ayant diverses couleurs d'origine, les temps de contact souhaitables pour obtenir une nuance particulière désirée.

### EXEMPLE 3 :

On cultive des *Impatiens balsamina* de semis. On récolte les parties aériennes que l'on pulvérise avec un broyeur. On obtient un broyat avec des dimensions moyennes de particules de 0,5 mm environ.

On prépare alors la composition suivante :
- Broyat 20 g
- Eau tiède qsp 100 g
- Hydroxyde de sodium q.s. pH 9,5

On applique cette composition sur des mèches de cheveux gris (contenant 90 % de cheveux blancs) à une température de 45°C et on maintient les cheveux à cette température pendant 30 minutes puis on rince les cheveux à l'eau et on les sèche.

On obtient une coloration brun jaunâtre moyen.

### EXEMPLE 4 :

Des broyats obtenus à partir de diverses parties *d'Impatiens balsamina* ont été préparés comme dans l'exemple 3, puis lyophilisés.

On a alors préparé les compositions suivantes :
- Lyophilisat broyé x grammes
- Eau tiède (30°C) qsp 100 grammes
- Acide chlorhydrique q.s. pH 5,5

La nature et la quantité de broyat, ainsi que les résultats de l'opération de teinture (effectuée comme décrit à l'exemple 3) sont indiqués ci-après.

| Parties de plantes | Quantité de broyat en grammes x = | Résultats |
|---|---|---|
| Tiges | 13,1 | Brun jaunâtre moyen |
| Fleurs | 7,9 | Brun jaunâtre moyen |
| Feuilles | 14,2 | Brun puissant |
| Parties aériennes | 11,6 | Brun jaunâtre puissant |

### EXEMPLE 5 :

On mélange 20 ml d'eau et 3 g de lyophilisat broyé. On applique la composition obtenue à température ambiante, sur cheveux gris, et on laisse agir la composition pendant une heure à température ambiante (21°C), puis on rince et sèche les cheveux.

Avec un broyat de fleurs, on obtient une coloration blond cuivré assez intense.

Avec un broyat de feuilles, on obtient une coloration analogue.

Avec un broyat de racines, on obtient une coloration blond légèrement doré.

### EXEMPLE 6 : Composition sous forme de pondre

On réalise la composition suivante :
- Poudre de feuilles *d'Impatiens balsamina* 55 g
- Gomme adragante 1 g
- Pectine 1 g
- Chlorure de distéaryl diméthyl ammonium 0,8 g
- Citrate de sodium trisodique qsp 100 g

Au moment de l'emploi de cette composition, on la mélange avec 1,5 fois son poids d'eau à 38°C.

On applique le mélange obtenu sur des cheveux blond clair et on laisse en contact pendant 45 minutes. Après rinçage, les cheveux présentent un reflet doré cuivré esthétique.

## Revendications

1. Composition colorante pour cheveux, comprenant comme agent colorant un broyat de plantes ou de parties de plantes de l'espèce *Impatiens balsamina*, sous la forme d'un broyat au moins partiellement déshydraté.

2. Compostion selon la revendication 1, caractérisée par le fait que la masse dudit broyat représente moins de 10 % de la masse des plantes fraîches ou parties de plantes fraîches de départ.

3. Composition selon l'une quelconque des revendications précédentes, caractérisée par le fait que ledit agent colorant est un lyophilisat broyé.

4. Composition selon l'une quelconque des revendications précédentes, caractérisée par le fait que lesdites parties de plantes sont choisies parmi les racines, les feuilles, les fleurs et les parties aériennes.

5. Composition selon l'une quelconque des revendications précédentes, caractérisée par le fait qu'elle contient en outre au moins un excipient et/ou au moins un agent de traitement des cheveux.

6. Composition selon la revendication 5, caractérisée par le fait que ledit excipient et/ou agent de traitement des cheveux est choisi parmi les agents épaississants, les agents tensioactifs, les agents antioxydants, les agents de pénétration, les agents séquestrants, les agents conservateurs, les tampons, les agents dispersants, les agents colorants, les agents filmogènes, les agents opacifiants et les parfums.

7. Composition selon la revendication 6, caractérisée par le fait que lesdits excipients et/ou agents sont ajoutés sous forme de poudre ou sous forme de solution d'imprégnation du broyat.

8. Composition selon l'une quelconque des revendications précédentes, caractérisée par le fait que ledit broyat a des dimensions de particules allant de 0,1 à 2000 µm, notamment de 0,5 à 750 µm, et en particulier de 1 à 500 µm.

9. Procédé de coloration des cheveux, caractérisé par le fait qu'il comprend les étapes consistant à :
a) obtenir une composition sous la forme d'une dispersion aqueuse contenant un broyat de plantes ou de parties de plantes de l'espèce *Impatiens balsamina*, dans un véhicule liquide approprié, ladite dispersion constituant une bouillie ayant une consistance suffisante pour enrober les cheveux et y adhérer sans s'écouler après l'application sur lesdits cheveux;
b) à mettre en contact les cheveux à colorer avec une composition telle qu'obtenue en a) ci-dessus ;
désirée c) et à maintenir ledit contact pendant un temps suffisant pour obtenir la coloration désirée.

10. Procédé selon la revendication 9, caractérisé par le fait que ladite dispersion est obtenue au départ d'une composition telle que définie dans l'une quelconque des revendications 1 à 8.

11. Procédé selon l'une quelconque des revendications 9 et 10, caractérisé par le fait que ledit temps de contact est dans la gamme de 5 minutes à 4 heures.

12. Procédé selon la revendication précédente, caractérisé par le fait que ledit temps de contact est compris entre 15 minutes et 1 heure.

## Patentansprüche

1. Färbende Zubereitung für die Haare, enthaltend als farbgebendes Mittel ein Zerkleinerungsgut von Pflanzen oder Pflanzenteilen der Species *Impatiens balsamina* in Form eines mindestens teilweise dehydrierten Zerkleinerungsgutes.

2. Zubereitung gemäß Anspruch 1, dadurch gekennzeichnet, daß die Masse des Zerkleinerungsgutes mindestens 10 % der Masse der ursprünglichen Frischpflanze oder der ursprünglichen Frischpflanzenteile entspricht.

3. Zubereitung gemäß einem der vorstehenden Ansprüche, dadurch gekennzeichnet, daß es sich bei dem farbgebenden Mittel um ein zerkleinertes Lyophilisat handelt.

4. Zubereitung gemäß einem der vorstehenden Ansprüche, dadurch gekennzeichnet, daß die Pflanzenteile ausgewählt sind unter den Wurzeln, Blättern, Blüten und luftzugänglichen Teilen.

5. Zubereitung gemäß einem der vorstehenden Ansprüche, dadurch gekennzeichnet, daß sie zusätzlich mindestens ein Excipiens und/oder mindestens ein Behandlungsmittel für die Haare enthält.

6. Zubereitung gemäß Anspruch 5, dadurch gekennzeichnet, daß das Excipiens und/ oder das Behandlungsmittel für die Haare ausgewählt ist unter den Verdickungsmitteln, den oberflächenaktiven Mitteln, den Antioxidantien, den Durchdringungsmitteln, den Sequestriermitteln, den Konservierungsmitteln, den Puffern, den Dispergiermitteln, den farbgebenden Mitteln, den filmbildenden Mitteln, den mattierenden Mitteln und den Parfümen.

7. Zubereitung gemäß Anspruch 6, dadurch gekennzeichnet, daß die Excipientien und/ oder Mittel in Form von Pulver oder in Form einer Imprägnierlösung für das Zerkleinerungsgut zugegeben werden.

8. Zubereitung gemäß einem der vorstehenden Ansprüche, dadurch gekennzeichnet, daß das Zerkleinerungsgut Teilchengrößen von 0,1 bis 2000 um, insbesondere von 0,5 bis 750 µm und insbesondere von 1 bis 500 um aufweist.

9. Färbeverfahren für die Haare, dadurch gekennzeichnet, daß es die Schritte, bestehend aus umfaßt:
(a) Erhalten einer Zubereitung in Form einer wäßrigen Dispersion, enthaltend ein Zerkleinerungsgut von Pflanzen oder Pflanzenteilen der Species *Impatiens balsamina* in einem geeigneten flüssigen Träger, wobei die Dispersion einen Brei mit einer zur Umhüllung der Haare und zum Anhaften ausreichenden Konsistenz darstellt, ohne nach dem Auftrag auf die Haare abzulaufen;
(b) in-Kontakt-bringen der zu färbenden Haare mit einer Zubereitung, wie sie oben in (a) erhalten wurde;
(c) und Erhalt des Kontakts für ausreichende Zeit, um die gewünschte Färbung zu erzielen.

10. Verfahren gemäß Anspruch 9, dadurch gekennzeichnet, daß die Dispersion ausgehend von einer Zubereitung, wie sie in einem der Ansprüche 1 bis 8 definiert ist, erhalten wird.

11. Verfahren gemäß einem der Ansprüche 9 und 10, dadurch gekennzeichnet, daß die Kontaktzeit im Bereich von 5 Minuten bis 4 Stunden liegt.

12. Verfahren gemäß dem vorstehenden Anspruch, dadurch gekennzeichnet, daß die Kontaktzeit zwischen 15 Minuten und 1 Stunde liegt.

## Claims

1. Hair dye composition including, as dyeing agent, a product of grinding plants or parts of plants of the species *Impatiens balsamina*, in the form of an at least partially dehydrated product of grinding.

2. Composition according to Claim 1, characterized in that the mass of the said product of grinding represents less than 10 % of the mass of the initial fresh plants or parts of fresh plants.

3. Composition according to any one of the preceding claims, characterized in that the said dyeing agent is a ground freeze-dried product.

4. Composition according to any one of the preceding claims, characterized in that the said parts of plants are chosen from roots, foliage, flowers and aerial parts.

5. Composition according to any one of the preceding claims, characterized in that it additionally contains at least one excipient and/or at least one hair-treatment agent.

6. Composition according to Claim 5, characterized in that the said excipient and/or hair-treatment agent is chosen from thickening agents, surface-active agents, antioxidants, penetrating agents, sequestering agents, preserving agents, buffers, dispersants, dyeing agents, film-forming agents, opacifying agents and perfumes.

7. Composition according to Claim 6, characterized in that the said excipients and/or agents are added in the form of powder or in the form of a solution for impregnating the product of grinding.

8. Composition according to any one of the preceding claims, characterized in that the said product of grinding has a particle size ranging from 0.1 to 2000 *µ*m, especially from 0.5 to 750 *µ*m, and in particular from 1 to 500 *µ*m.

9. Hair dyeing process characterized in that it includes the stages consisting in:
a) obtaining a composition in the form of an aqueous dispersion containing a product of grinding plants or parts of plants of the species *Impatiens balsamina*, in an appropriate liquid carrier, the said dispersion forming a pulp which has a consistency that is sufficient to coat the hair and to adhere thereto without running after the application to the said hair;
b) bringing the hair to be dyed into contact with a composition as obtained in a) above;
c) and maintaining the said contact for a sufficient period to obtain the desired colour.

10. Process according to Claim 9, characterized in that the said dispersion is obtained from a composition as defined in any one of Claims 1 to 8.

11. Process according to either of Claims 9 and 10, characterized in that the said contact time is in the range from 5 minutes to 4 hours.

12. Process according to the preceding claim, characterized in that the said contact time is between 15 minutes and 1 hour.
